(19) Europäisches Patentamt
European Patent Office
Office européen des brevets

(11) EP 2 819 647 B1

# (12) FASCICULE DE BREVET EUROPEEN

(45) Date de publication et mention de la délivrance du brevet:
**02.08.2017 Bulletin 2017/31**

(21) Numéro de dépôt: **13720033.3**

(22) Date de dépôt: **26.02.2013**

(51) Int Cl.:
***A61K 9/06*** (2006.01) ***A61K 47/32*** (2006.01)
***A61K 9/00*** (2006.01) ***A61J 1/20*** (2006.01)
***A61K 9/10*** (2006.01) ***A61K 49/04*** (2006.01)

(86) Numéro de dépôt international:
**PCT/IB2013/051536**

(87) Numéro de publication internationale:
**WO 2013/128373 (06.09.2013 Gazette 2013/36)**

## (54) ASSOCIATION DE POLY(N ACRYLOYL GLYCINAMIDE) AVEC AU MOINS UN PRINCIPE ACTIF

POLY (N ACRYLOYL GLYCINAMIDE) IN ASSOZIATION MIT WENIGSTENS EINEM WIRKSTOFF

POLY (N ACRYLOYL GLYCINAMIDE) ASSOCIATED WITH AT LEAST AN ACTIVE AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.02.2012 FR 1251765**

(43) Date de publication de la demande:
**07.01.2015 Bulletin 2015/02**

(73) Titulaires:
- **Centre National de la Recherche Scientifique 75016 Paris (FR)**
- **Institut Regional du Cancer de Montpellier F-34298 Montpellier Cedex 5 (FR)**
- **Université Montpellier 1 34967 Montpellier Cedex 2 (FR)**

(72) Inventeurs:
- **BOUSTTA, Mahfoud F-34570 Pignan (FR)**
- **COLOMBO, Pierre-Emmanuel F-34830 Clapiers (FR)**
- **VERT, Michel F-34170 Castelnau-le-Lez (FR)**

(74) Mandataire: **Nony
11 rue Saint-Georges
75009 Paris (FR)**

(56) Documents cités:
**EP-A1- 1 312 627 JP-A- 2008 220 868**
**US-B2- 7 976 728**

- **KUILIN DENG ET AL: "A pH/Thermo-responsive injectable hydrogel system based on Poly(N-acryloylglycine) as drug carrier", IRANIAN POLYMER JOURNAL, vol. 20, no. 3, 2011, pages 185-194, XP002683974,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention est relative à des formulations galéniques formées à partir de polymères intelligents, et plus particulièrement de polymères thermo-réactifs possédant en solution aqueuse une température critique supérieure de solubilité ou TCSS (UCST ou Upper Critical Solution Température en anglais) compatible avec la formation réversible d'une phase gélifiée (gel) par refroidissement d'une solution chaude lorsqu'ils sont mis en contact avec le corps humain ou animal, en particulier lors de la phase de refroidissement.

Par « polymère intelligent » ou « polymère adaptatif » ou « polymère réactif », on entend un polymère capable de répondre à un stimulus extérieur par un changement de propriétés. Ce stimulus extérieur peut par exemple être la température, la pression, le pH, la force ionique, la lumière ou un stimulus mécanique, électrique ou magnétique. En langue anglaise, un tel type de polymère est aussi couramment appelé « stimuli-responsive polymer » ou « smart polymer ». Le plus souvent, les chaînes polymères voient leur conformation se modifier en réponse au stimulus, conférant le changement de propriétés. Cette modification de conformation est généralement assortie à une modification chimique, ces deux modifications étant simultanées, réversibles (association par liaison hydrogène, ionique ou dipolaire, solvatation ou désolvatation) ou irréversible (isomérie chimique), et le plus souvent à caractère coopératif

Il est connu d'utiliser ou, plus généralement, de seulement proposer d'utiliser certains de ces polymères intelligents dans le domaine biomédical. On peut par exemple se rapporter à l'article « Smart Polymers and Their Applications as Biomaterials », Aguilar et al., Topics in Tissue Engineering, 2007, Vol. 3.

Parmi lesdits polymères intelligents déjà employés dans le domaine de la libération de principes actifs, ont été décrits en particulier des polymères sensibles aux changements de pH (« pH-sensitive polymers »). Ont également été décrits des polymères sensibles aux changements de température (« temperature sensitive polymers ») et plus particulièrement les polymères relevant d'un phénomène de température critique inférieure de solubilité ou TCIS, (LCST ou Lower Critical Solution Temperature en anglais) correspondant à une phase solution à basse température en dessous de la TCIS et à une phase condensée plus ou moins solide au dessus.

Il existe également des systèmes de type TCSS, caractérisés par une phase condensée plus ou moins solide à basse température en dessous de la TCSS et à une phase liquide au dessus. Dans ces systèmes, la phase condensée est constituée d'un gel hydrophile et gonflé d'eau, contrairement aux phases condensées de type TCIS qui sont largement désolvatées avec un caractère hydrogel (présence d'eau interne) beaucoup moins important au dessus de la TCIS.

Parmi les systèmes de type TCIS, figurent les poly(N-isopropyl acrylamide)s, connus sous les abréviations PNIPAM, PNIPAAm ou polyNIPAM, ou encore les copolymères à blocs de type dibloc (A-B) ou tribloc (A-B-A ou B-A-B) où A est un segment poly(oxyde d'éthylène) et B un segment poly(acide lactique), ou encore les copolymères dibloc ou tribloc où A est un segment poly(oxyde d'éthylène) et B un segment de poly(oxyde de propylène). Parmi les composés de type TCIS, le poly(N-isopropyl acrylamide) a été particulièrement étudié en vue d'applications biomédicales.

Le poly(N-isopropyl acrylamide) présente une température de transition sol-gel réversible aux environs de 32°C en solution aqueuse compatible avec les organismes animaux et humains. Cette température variable peut être modifiée par copolymérisation avec des co-monomères, notamment ceux introduisant des entités hydrophobes ou ioniques ou encore hydrophiles.

On connaît par ailleurs le poly(N-acryloyl glycinamide), connu sous les abréviations PAG et PNAG, qui a en tout premier lieu été décrit par Haas dans les années 60 dans une série de publications entre 1964 («Thermally reversible homopolymer gel systems », Hass et al., Journal of Polymer Science, Part B, 1964, Vol. 2, 12:1095) et 1970 (« Differential thermal analysis of thermally reversible gels», Haas et al., Anal. Calorimetry, Proc. Symp., 1970, 2:211).De plus, on connaît de US 2009/0053276 une composition injectable qui contient des particules d'hydrogel thermo-sensible, y compris des particules formées à partir de polymères présentant une TCSS (UCST). Toutefois, les poly(N-acryloyl glycinamide)s n'y sont pas décrits et le seul phénomène recherché est l'expansion des particules par réchauffement à la température du corps humain.

On connaît également de Kuilin Deng et al. (Iranian Polymer Journal, 2011, Vol.20, 3:185) un polymère présentant une TCSS (UCST) et donc injectable en solution. Toutefois, les poly(N-acryloyl glycinamide)s n'y sont pas non plus décrits et l'utilité du polymère décrit pour la délivrance de principe actif par injection n'est pas adaptée aux conditions thermiques de la voie parentérale.

Certains auteurs ont également étudié les propriétés de PAGs obtenus par la méthode de polymérisation Reversible Addition-Fragmentation Transfer (RAFT). Cependant, les PAGs obtenus par cette méthode de polymérisation ne possèdent pas de température de transition gel-sol. L'absence de transition gel-sol pour des PAGs obtenus par polymérisation RAFT est d'ailleurs souligné dans l'article « Well-defined uncharged polymers with a sharp UCST in water and in physiological milieu », Glatzel S. et al., Macromolecules, 2011, 44:413.

Les inventeurs ont mis en évidence que l'utilisation du poly(N-acryloyl glycinamide) possédant une température de transition gel-sol dans une formulation galénique comprenant un principe actif dans un but d'application topique en vue d'une libération et une diffusion dudit principe actif localement, présente de nombreux avantages alors même que le poly(N-acryloyl glycinamide) n'avait jamais été proposé dans cette application.

Ainsi, la présente invention décrit l'association de poly(N-acryloyl glycinamide) avec au moins un principe actif et/ou au moins un produit visible en imagerie médicale, dans un milieu aqueux physiologiquement acceptable.

La présente invention a ainsi pour objet l'association de poly(N-acryloyl glycinamide) avec au moins un principe actif et/ou au moins un produit visible en imagerie médicale, dans un milieu aqueux physiologiquement acceptable, ladite association présentant une température de transition gel-sol comprise entre 30 et 60°C, et étant sous forme liquide au dessus de la température de transition gel-sol et sous forme gel en dessous de la température de transition gel-sol, pour son utilisation dans le traitement local post-opératoire ou per-opératoire,
pour son utilisation dans le traitement des plaies, caractérisée en ce qu'elle est directement appliquée sur la plaie ou *via* tout dispositif de protection, ou
pour son utilisation dans le suivi post-opératoire en imagerie médicale.

Elle a également pour objet l'association telle que définie ci-dessus, dans laquelle ledit poly(N-acryloyl glycinamide) présente une masse molaire moyenne comprise entre 10 000 et 1 000 000 g/mol, en particulier entre 30 000 et 600 000 g/mol, voire entre 40 000 et 200 000 g/mol.

L'association selon l'invention présente une température de transition gel-sol comprise entre 30 et 60 °C, et de préférence entre 38 et 50°C, par exemple mesurée par la méthode du tube renversé.

L'association selon l'invention est sous forme liquide au dessus de la température de transition gel-sol et sous forme gel en dessous de la température de transition gel-sol.

Plus particulièrement, selon le site d'application considéré, on choisit une association présentant une température de transition gel-sol supérieure à la température du site d'application. Par exemple, si le site d'application considéré est la cavité intrapéritonéale, on choisira une association possédant une température de transition gel-sol supérieure à 37°C. De même, on choisira une association possédant une température de transition gel-sol supérieure à 30°C si le site d'application considéré est la peau.

Selon un autre aspect, la présente invention également l'association telle que définie ci-dessus, pour son utilisation dans la délivrance de principes actifs.

Selon encore un autre aspect, la présente invention décrit l'utilisation du poly(N-acryloyl glycinamide) présentant une masse molaire moyenne comprise entre 10 000 et 1 000 000 g/mol, en particulier entre 30 000 et 600 000 g/mol, voire entre 40 000 et 200 000 g/mol, pour la préparation d'une solution aqueuse gélifiable comprenant un principe actif et/ou un produit visible en imagerie médicale.

Au regard des propriétés de l'association sous forme de gel conforme à la présente invention et en particulier de sa bonne adhérence aux tissus, une des applications plus particulièrement visées dans le cadre de la présente invention concerne la délivrance de principes actifs destinés au traitement antitumoral local per-opératoire, notamment complémentaire d'une chirurgie de réduction tumorale, par exemple d'un cancer abdominal en carcinose péritonéale.

En effet, cette application particulière répond au besoin du chirurgien cancérologue de disposer d'un traitement post-opératoire fiable. En effet, lorsque le chirurgien vient de procéder à l'ablation d'une tumeur, il se trouve confronté au risque d'avoir laissé quelques cellules tumorales susceptibles de régénérer la tumeur ou pire de migrer pour essaimer des métastases dispersées. Actuellement, le chirugien cancérologue introduit une solution d'un agent antitumoral dans la cavité intrapéritonéale qu'il remplit. A la fermeture du site opératoire, le liquide peut s'épancher, ce qui est un inconvénient majeur. Pour éviter l'épanchement du liquide, le chirurgien l'aspire souvent après un séjour relativement court.

L'utilisation du poly(N-acryloyl glycinamide) permet de résoudre les inconvénients précités et en particulier d'obtenir un système de libération suffisamment adhérent aux tissus. Par ailleurs, l'utilisation d'une solution aqueuse gélifiable, conforme à la présente invention, présente l'avantage de permettre d'enrober ou d'imprégner une tumeur ou des organes tels que les organes de la cavité intrapéritonéale, notamment lors d'une opération chirurgicale.

Ainsi, la présente invention a plus particulièrement pour objet l'association de poly(N-acryloyl glycinamide) avec au moins un agent antitumoral, dans un milieu aqueux physiologiquement acceptable.

Elle a également pour objet l'association telle que définie ci-dessus, utile pour le traitement antitumoral local per-opératoire des patients ayant subi une chirurgie de réduction tumorale. Par exemple, on peut citer l'ablation d'une tumeur de la cavité intrapéritonéale.

La présente invention a également pour objet l'association selon l'invention pour son utilisation dans le traitement des plaies, caractérisée en ce qu'elle est directement appliquée sur la plaie ou *via* tout dispositif de protection, notamment un pansement, par exemple par imprégnation dudit dispositif de protection par un gel formé à partir d'une solution chaude.

L'association selon l'invention est utile pour le traitement des animaux, et en particulier pour le traitement des humains.

En particulier, l'association selon l'invention est utile dans le domaine vétérinaire.

La présente invention présente de plus l'avantage de fournir un système pouvant se réticuler *in situ,* sans faire appel à des réactions de chimie covalente.

L'association selon l'invention peut ainsi être injectée sous forme liquide et gélifiable *in situ* en un temps court afin de ne pas permettre de diffusion ou de dispersion au delà du site d'administration. Ainsi, la gélification survient quasi instantanément.

En particulier, l'association selon l'invention gélifie dans un temps inférieur à 5 minutes, de préférence inférieur à 3 minutes, et mieux inférieur à 2 minutes.

La température de gélification d'une association selon l'invention est insensible à la présence de sel, notamment de NaCl 0,15N, et donc à la force ionique qui peut être imposée par un milieu parentéral.

L'injection de l'association peut se faire à l'aide d'une seringue conventionnelle et d'aiguilles de petite ouverture.

Par ailleurs, la forme gel de l'association selon l'invention est biocompatible et stérilisable.

Ainsi, l'association conforme à la présente invention présente également l'avantage d'être stable dans le temps et de permettre son stockage jusqu'à son application.

Le stockage peut ainsi être effectué sous forme gel puis réchauffé pour sa mise en solution avant injection.

La composition peut alors avantageusement être stockée au réfrigérateur, au congélateur ou encore sous forme lyophilisée.

Par ailleurs, la stérilisation peut être faite par ultrafiltration des formes solution à travers des ultrafiltres stérilisant (par exemple 0,22 $\mu$m).

Par ailleurs, l'association selon la présente invention est particulièrement avantageuse car l'hydrogel, une fois constitué avec le milieu aqueux, reste stable si un excès de milieu aqueux est ajouté. Cette stabilité est importante plus particulièrement dans la délivrance de principes actifs destinés au traitement antitumoral local per-opératoire, notamment après chirurgie de réduction tumorale car *in situ,* l'hydrogel est soumis aux fluides et exsudats voisins post-opératoires, plus ou moins abondants selon le site.

Comme cela apparaîtra de façon plus détaillée dans la suite de la description, la mise en oeuvre de l'invention présente également un avantage sur le plan de l'étendue des formulations galéniques possibles.

## Figures

La figure 1, en lien avec l'exemple 1, représente la variation du degré de polymérisation moyen en nombre $DP_n$ des PAG obtenus en fonction de différentes concentrations en 2-propanol (agent de transfert : AT) avec une concentration en monomère M constante.

La figure 2, en lien avec l'exemple 1, décrit la gamme de températures critiques selon la concentration en poly(N-acryloyl glycinamide).

Plus précisément, la figure 2 représente une variation des températures de séparation de phase (transition gel-sol) en fonction de la concentration faible en PAG observée pour des PAG de différentes masses molaires moyennes (du haut vers le bas : masses décroissantes).

La figure 3, en lien avec l'exemple 1, représente la gamme de températures critiques selon la masse molaire moyenne du poly(N-acryloyl glycinamide).

Plus précisément, la figure 3 représente une variation des températures de séparation de phase (transition gel-sol) en fonction de la masse moléculaire Mn viscosimétrique des PAG pour différentes concentrations en poly(N-acryloyl glycinamide).

La figure 4, en lien avec l'exemple 6, représente la courbe de libération d'acétate de cobalt dans le poly(N-acryloyl glycinamide) de masse molaire moyenne égale à 170000 g/mol, en fonction du temps.

La figure 5, en lien avec l'exemple 6, représente la courbe de libération du poly(acrylate de sodium) dans le poly(N-acryloyl glycinamide) de masse molaire moyenne égale à 170000 g/mol, en fonction du temps.

La figure 6, en lien avec l'exemple 6, représente la courbe de libération de l'oligomère de poly(tyrosine-formaldéhyde) dans le poly(N-acryloyl glycinamide) de masse molaire moyenne égale à 170000 g/mol, en fonction du temps.

## Définitions

Par « gel-sol », on entend une contraction des termes « gélification-solution ».

Par « hydrogel », on entend une matrice tridimensionnelle (réticulée physiquement ou chimiquement) gonflée par de l'eau en milieu aqueux.

Par « biodégradable », on entend une substance ou un matériau qui se décompose sous l'action de cellules vivantes isolées ou impliquées dans un tissu ou dans un organe.

Par « biorésorbable », on entend une substance ou un matériau qui se dégrade enzymatiquement ou hydrolytiquement et pour lequel on a la preuve que les produits de dégradation sont intégrés en biomasse et/ou éliminés de l'organisme par la voie aérienne après métabolisation et/ou par filtration rénale.

Par « quantité suffisante », on entend une quantité minimale nécessaire pour parvenir à l'effet attendu, par exemple, la reconstitution d'un mélange présentant les propriétés visées pour l'application considérée.

Par « support galénique pour application topique », on entend un support capable de véhiculer et/ou d'appliquer

un principe actif sur un site à traiter, par exemple par formation d'un gel hébergeant temporairement ledit principe actif.

Par « UCST » ou « TCSS » ou « température critique supérieure de solubilité », on entend la température maximale au dessus de laquelle un système polymère-milieu aqueux se présente sous forme d'une phase liquide homogène. Les systèmes polymère-milieu aqueux présentant une température critique supérieure de solubilité (TCSS) sont sous forme gélatineuse sous la TCSS qui disparaît lorsqu'on élève la température au dessus de cette température critique. Ces polymères doivent leur insolubilité, à température inférieure à leur TCSS, à la présence d'interactions intra- et intermoléculaires réversibles telles que ioniques ou électrostatiques associés.

Plus précisément, la température critique supérieure de solubilité (TCSS) d'un mélange polymère-solvant est la température au dessus de laquelle les composants sont miscibles et le système est une phase liquide homogène à l'oeil nu quelle que soit la composition du mélange. Dans la pratique, un tel système polymère-solvant peut être caractérisé par une transition de phase gel-sol réversible qui dépend de la pression, de la masse molaire moyenne et de la polymolécularité ou distribution des macromolécules de masses molaires différentes ("Pure and Applied Chemistry", 2004, 76:1985 ; Definition of terms related to polymer blends, composites, and multiphase polymeric materials, IUPAC Recommendations, 2004, page 1999).

Par « température de transition gel-sol », on entend la température au dessus de laquelle un polymère donné présentant une TCSS en solution aqueuse passe réversiblement d'une phase hydrogel (gel gonflé de plus ou moins d'eau) à une phase liquide et devient donc soluble dans le milieu aqueux. La température de transition gel-sol réversible dépend des conditions de mise en oeuvre, par exemple un cyclage gel-sol unique ou multiple, la vitesse de refroidissement ou le volume à refroidir.

Dans le texte, les expressions « compris entre ... et ... », « allant de ... à ... » et « variant de ... à ... » sont équivalentes et entendent signifier que les bornes sont incluses, sauf mention contraire.

Dans la description, l'expression « au moins un ... », doit être comprise comme « un ou plusieurs ... ».

Sauf indication contraire, l'expression « comportant/comprenant un(e) » doit être comprise comme « comportant/comprenant au moins un(e) ».

**<u>Polymère</u>**

Le poly(N-acryloyl glycinamide) ou PAG ou poly(NAGA) est connu en soi bien qu'apparemment non disponible commercialement.

En réalité, on désigne par poly(N-acryloyl glycinamide) ou ses abréviations une famille de composés macromoléculaires qui diffèrent par leurs masses molaires moyennes en masse et en nombre, et par la dispersité de celles-ci traduite par l'indice de polymolécularité ou encore la polydispersité définis par le rapport de la masse molaire moyenne en masse sur la masse molaire moyenne en nombre.

Les macromolécules de type PAG présentent la formule (I) suivante :

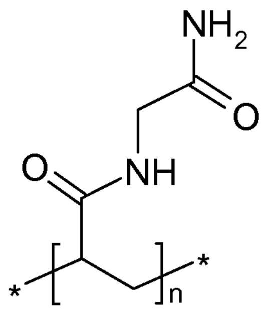

(I)

Dans le cadre de la présente invention, la masse molaire moyenne du PAG est de préférence comprise entre 10 000 et 1 000 000, plus particulièrement entre 30 000 et 600 000, voire entre 40 000 et 200 000 g/mol.

Selon un mode de réalisation particulier, la masse molaire moyenne est évaluée par viscosimétrie, par exemple comme détaillé dans l'exemple 1 ci-après.

A l'image des exemples, les valeurs viscosimétriques peuvent êtres mesurées par un viscosimètre de type Ubbelohde.

Ce polymère en solution est un polymère thermosensible. Dans le cas du PAG, la réticulation thermo-dépendante se fait par la formation d'un réseau de liaisons hydrogène entre les groupes NH et C=O présents dans les chaînes pendantes de type glycinamide riches en groupes fonctionnels interactifs. On dit que les hydrogels de PAG dépendent d'un phénomène de température critique supérieure de solubilité (TCSS ou UCST ou solution à haute température et gel à basse température).

Les hydrogels de PAG sont parfois appelés « gélatine artificielle ».

Plus particulièrement, un polymère PAG, mélangé à un milieu aqueux peut conduire à une solution (sol) ou hydrogel (gel) selon la température dudit milieu.

L'intérêt de base de ce polymère en tant que source d'hydrogel intelligent est que, mélangé à un milieu aqueux, il peut conduire à une solution (sol) ou un hydrogel (gel) selon la température et que la transition gel-sol peut être amenée dans la zone de température d'un organisme animal de telle sorte qu'administré sous forme sol à une température supérieure mais compatible avec celle de l'organisme en question (humain ou animal), il se gélifie lorsque le sol administré revient à cette dernière température.

Dans le cadre de la présente invention, on peut également considérer les copolymères de poly(N-acryloyl glycinamide) avec divers monomères tels que décrit dans « Synthetic thermally reversible gel systems », Haas et al., Journal of Polymer Science, Polymer Physics Edition, II, 1967, Vol. 5, 5:915. De tels monomères peuvent être en particulier choisis de telle sorte que les solutions de ces copolymères satisfassent aux conditions imposées par les organismes animaux et/ou humains.

L'homme de l'art est à même de synthétiser le polymère convenant à l'invention selon ses connaissances générales. La polymérisation (et la copolymérisation éventuelle) peut être amorcée en masse ou en solution dans un solvant organique par des composés générateurs de radicaux libres. Les amorceurs de type anionique et cationique peuvent également être utilisés, à condition que cela soit propice à l'obtention de PAG possédant une température de transition gel-sol.

Par exemple, le polymère convenant à l'invention peut être préparé selon le protocole décrit dans les articles publiés par Haas, notamment dans « Synthetic thermally reversible gel systems », Haas et al., Journal of Polymer Science, IV, Part A-1, Polymer Chemistry, 1970, Vol. 8, 5:1213.

Dans le cadre de la présente invention, la polymérisation par amorçage radicalaire par décompositions chimiques sera privilégiée avec l'utilisation d'amorceurs générant des radicaux en température tels que le peroxyde de benzoyle, le peroxyde de tertio-butyle, les composés diazoïques tels que l'azo-bis-isobutyronitrile, les composés peroxygénés tels que les persulfates ou l'eau oxygénée, les systèmes redox tels que l'oxydation de $Fe^{2+}$ ou de $Ce^{4+}$, les mélanges persulfate/sodium-metabisulfite, ou l'acide ascorbique/eau oxygénée ou encore les composés clivables photochimiquement ou par radiations ionisantes par exemple par UV ou par rayonnements beta et gamma.

Selon un mode de réalisation particulier, le polymère convenant à l'invention est préparé par une méthode de polymérisation radicalaire.

Afin de maîtriser les masses molaires moyennes, comme cela est plus particulièrement illustré dans l'exemple 1, un agent de transfert peut être utilisé.

Par exemple, l'obtention de masses molaires moyennes plus ou moins élevées peut se faire par l'usage d'agents de transfert tels que décrit dans Haas et al., Journal of Polymer Science, 1964, Vol. 2, 12:1095 et plus récemment dans « Well-defined synthetic polymer with a protein-like gélation behavior in water », Glatzel et al., Chemical Communications, 2010, 45, 4517.

L'agent de transfert a pour but d'arrêter la croissance des chaînes macromoléculaires en cours de formation par addition de molécules monomères et d'amorcer de nouvelles chaînes, ce qui permet de limiter les masses moléculaires finales, voire de les contrôler.

Dans le cadre de la présente invention, un agent de transfert peut être plus particulièrement choisi parmi les thiols tels que le dodécylthiol $C_{12}H_{25}SH$ ou l'acide thioglycolique $HSCH_2COOH$, les dérivés halogénés tels que $CHCl_3$ ou les dérivés bromés, ou les alcools tels que l'isopropanol, comme indiqué dans US 3,396,030.

On peut également obtenir des PAGs de masses molaires moyennes différentes à partir d'un PAG par fractionnement en triangle à l'aide d'un mélange solvant-non-solvant par exemple selon une méthode classique en science des polymères, bien connue de l'homme de l'art.

## Température de transition gel-sol

Dans le cadre de la présente invention, la température de transition gel-sol se situe avantageusement à une température supérieure à la température corporelle et telle que le système soit sous forme solution (sol) à une température supérieure à la température corporelle mais inférieure à la température maximum que peuvent supporter les tissus, à savoir 60°C, de préférence 50°C, et soit sous une forme hydrogel (gel) à la température corporelle du site d'administration, généralement 37°C chez l'homme, valeur différente chez l'animal, et de toute façon, entre 35°C et 42°C.

Ainsi, l'association selon l'invention présente une température de transition gel-sol comprise entre 30 et 60°C, voire entre 38 et 50°C, et mieux entre 35 et 45°C, par exemple mesurée par la méthode du tube renversé.

La température de transition gel-sol est facilement adaptable au besoin en matière de température du site à traiter. En particulier, il est possible de moduler la température de transition gel-sol à travers la variation de divers paramètres.

En effet, la température de transition gel-sol d'un mélange donné comprenant un polymère donné dépend de

facteurs macromoléculaires tels que la masse molaire moyenne, la distribution des masses molaires, la présence de co-monomères éventuels, de la concentration du polymère dans la solution aqueuse, de la salinité, ainsi que de la présence d'additifs, notamment de composés hydrophobes, y compris les principes actifs.

Par exemple, la température de transition gel-sol est notamment dépendante à la fois de la concentration et de la masse molaire moyenne du polymère. Le fait d'augmenter une de ces deux variables d'ajustement a pour effet d'accroître les interactions intra- et interchaînes augmentant ainsi la température de transition gel-sol.

En pratique, on peut avantageusement utiliser ces deux paramètres pour obtenir un polymère conforme aux exigences en termes de température de transition gel-sol, comme illustré dans l'exemple 1.

Pour ce qui est du lien entre la température critique de transition gel-sol, la masse molaire moyenne et la concentration du polymère dans la solution aqueuse, on peut en particulier se rapporter à la figure 1 qui montre la gamme de températures de transition gel-sol selon la masse molaire moyenne et la concentration en polymère.

La présence de sel, notamment du milieu constituant le liquide physiologique à force ionique et pH compatible et imposé avec les fluides biologiques, n'altère pas de manière significative le comportement d'un PAG donné, qui est un polymère neutre, observé dans l'eau, notamment l'aptitude à former un gel.

La présence d'un composé hydrophobe ou hydrophile dans une formulation peut modifier la température de transition gel-sol observée en leur absence. Toutefois, il est possible de compenser l'effet en jouant sur les paramètres propres au polymère, tels que la masse molaire moyenne et la concentration.

Méthodes de mesure de la température de transition gel-sol

La valeur de la température de transition gel-sol dépend de la technique utilisée pour sa détermination.

Par exemple, on peut utiliser la méthode du tube renversé, la méthode de la chute de bille ou la mesure de viscosité dynamique.

Dans le cadre de la présente invention, la température de transition gel-sol peut être mesurée par la méthode du tube renversé.

Le principe de la méthode du tube renversé consiste à gélifier une solution à base de PAG dans un tube en verre équipé d'un thermomètre fixé par un bouchon, à incliner le tube tête en bas et à monter puis descendre progressivement la température de l'ensemble en notant les températures auxquelles la matière commence à couler par simple gravité lors de l'augmentation et/ou lorsque le milieu se fige à la décroissance.

Plus particulièrement, le poly(N-acryloyl glycinamide) séché est introduit dans un tube en verre de 20 $cm^3$ avec de l'eau déionisé. Le mélange est placé dans un bain d'eau à 80°C jusqu'à dissolution totale. La solution est ensuite refroidie à température ambiante et gélifiée.

Le système est ramené à 80°C pendant 5 minutes, puis refroidi à 0°C à une vitesse de 40°C/min, laissé à 0°C pendant 30 minutes puis chauffé à 80°C à une vitesse de 4°C/min.

Une première température de transition gel-sol est déterminée lorsque le gel commence à s'écouler le long de la paroi du tube. Ce cycle est répété 3 fois pour donner quatre températures gel-sol.

Une reproductibilité de ± 2°C de la température de transition gel-sol est généralement observée après le second cycle.

**Principe actif et produit visible en imagerie médicale**

Principes actifs

A titre de principes actifs pouvant convenir à la présente invention, on peut en particulier citer les agents antitumoraux, les antibiotiques, les analgésiques, les anti-inflammatoires, les facteurs de croissance et les antiseptiques.

Parmi les antibiotiques, conviennent notamment à l'invention ceux de la classe des aminoglycosides, tels que la gentamicine ou la kanamycine ; des ansamycines ; des carbacéphèmes ; des carbapénèmes ; des céphalosporines première génération, tels que la céfadroxile ou la céfazoline ; des céphalosporines seconde génération, tels que le céfaclor ou le céfamandole ; des céphalosporines troisième génération, tels que la céfixime ; des céphalosporines quatrième génération ; des céphalosporines cinquième génération, tels que la céftobiprole ; des glycopeptides ; des lincosamides ; des lipopeptides ; des macrolides ; des monobactames ; des nitrofuranes ; des pénicillines, tels que l'amoxicilline ou l'ampicilline ; des combinaisons de pénicillines ; des polypeptides ; des quinolones, tels que la ciprofloxacine ou l'ofloxacine ; des sulfonamides, tels que la sulfadiazine ou la sulfadiazine argentique ; des tétracyclines, tels que l'oxytétracycline ou la tétracycline ; des antibiotiques utiles contre les mycobactéries ; ou encore la métronidazole ou le thiamphénicole.

Parmi les analgésiques convenant plus particulièrement à l'invention, on peut notamment citer la buprenorphine, le fentanyl ou l'ibuprofène.

Les antiseptiques convenant à l'invention peuvent plus particulièrement être choisis parmi l'hexomédine, les

sels d'argent et l'iode.

Parmi les facteurs de croissance convenant à l'invention, peuvent être tout particulièrement cités les FGFa et FGFb, les VGF et les GRF.

De préférence, on peut notamment citer tout principe actif à action antitumorale. Parmi ces principes actifs, on peut citer plus particulièrement la doxorubicine, le paclitaxel, le topotécan, et l'irinotécan.

On peut également citer les composés radioactifs pour la radiothérapie interne, en particulier les grains d'Iode 125, notamment utilisés dans le traitement du cancer de la prostate.

Par ailleurs, les principes actifs précités peuvent le cas échéant être formulés sous forme de micro- ou nano-particules préférentiellement biorésorbables pour être piégées dans le gel. De même, le principe actif peut se présenter sous la forme de conjugués polymère vecteur-principe actif, classiquement nommés pro-drogue macromo léculaire.

Plusieurs principes actifs de nature différente peuvent être administrés simultanément grâce à la solution conforme à la présente invention. Cette polyvalence est particulièrement intéressante pour les sites tumoraux multirésistants. L'intérêt est facilement étendu au piégeage temporaire de peptides, de protéines, de gènes, et de molécules qui en général posent un problème en raison de leur solubilité exclusive dans les milieux aqueux et qui, dans le cas de systèmes particulaires matriciels ou capsulaires, amènent souvent une libération trop rapide et inadaptée et mobilisent généralement une quantité de matière étrangère au milieu vivant importante par rapport à celle impliquée dans les formulations à base de PAG.

<u>Produits visibles en imagerie médicale</u>

L'association selon l'invention peut également comprendre au moins un produit visible en imagerie médicale.

A titre de produit visible en imagerie médicale, on peut tout particulièrement citer les opacifiants, tel qu'un oxyde ou un sel minéral, par exemple le sulfate de baryum ou l'oxyde de zirconium pour permettre un suivi par rayon X, des particules magnétiques ou des nanoparticules paramagnétiques pour perturbation par un champ magnétique.

On peut également citer les agents fluorescents ou les émetteurs de radiations ionisantes, pour rendre l'hydrogel visible par les techniques appropriées correspondantes.

De préférence, le produit visible en imagerie médicale est choisi parmi un opacifiant, un agent fluorescent ou un émetteur de radiations ionisantes.

Selon une variante de réalisation, l'association selon l'invention comprend du poly(N-acryloyl glycinamide), au moins un principe actif et au moins un produit visible en imagerie médicale, dans un milieu aqueux physiologiquement acceptable.

**<u>Milieu aqueux physiologiguement acceptable</u>**

Le milieu aqueux physiologiquement acceptable selon l'invention est de préférence préparé à base d'un liquide physiologique.

Un liquide physiologique est un liquide isotonique au sang, c'est-à-dire présentant la même osmolarité que les principaux fluides corporels, en particulier le sang, soit environ 300 mosm/L et généralement tamponné à pH = 7,4.

Un tel liquide peut également être nommé solution physiologique ou sérum physiologique.

Ce liquide est généralement composé d'eau distillée et de chlorure de sodium NaCl dilué à 9 pour 1000, soit une solution à 0,9 % de poids/volume de NaCl, soit 9 g/L.

Le liquide physiologique peut également contenir du KCl, du $CaCl_2$, ou du $MgSO_4$.

Par « milieu physiologiquement acceptable », on entend plus généralement un milieu dénué de toxicité et compatible avec l'injection et/ou l'application de l'association considérée selon l'invention. En particulier, on entend un milieu compatible avec les fluides, tissus et cellules du site d'application considéré.

**<u>Association selon l'invention</u>**

Comme déjà exposé ci-dessus, l'association selon l'invention comprend du poly(N-acryloyl glycinamide) et au moins un principe actif et/ou au moins un produit visible en imagerie médicale, dans un milieu aqueux physiologiquement acceptable.

Selon un mode de réalisation particulier, le poly(N-acryloyl glycinamide) est présent dans le milieu aqueux dans une teneur en matière sèche comprise entre 0,5 et 20 % en poids par rapport au poids total dudit milieu, en particulier dans une teneur comprise entre 1 et 10% en poids, voire plus particulièrement entre 2 et 8% en poids.

L'association selon l'invention peut également comprendre des additifs, notamment des hydrophobisants tel qu'un lipide, un acide gras ou une amine grasse.

Dotés d'un squelette polyacrylique, les PAG ne sont pas intrinsèquement dégradables et/ou biodégradables, et encore moins biorésorbables. Toutefois, les quantités administrées sont généralement très faibles et les risques

d'accumulation sont eux-mêmes faibles car les teneurs en polymère qui sont administrées restent très faibles, par exemple entre 2 et 8%, en poids par rapport au poids total de la composition considérée, et ce afin de satisfaire la gamme de température de transition gel-sol visée dans le cadre de la présente demande et imposée par le milieu vivant.

Par ailleurs, il est possible d'obtenir un PAG présentant des propriétés améliorées de biodégrabilité par ajout d'un additif insoluble légèrement basique tel que MgO, CaO. Toutefois, il est possible d'altérer les chaînes latérales en présence de particules d'un composé insoluble plus ou moins basique (par exemple CaO, $Ca(OH)_2$, MgO, $Mg(OH)_2$, ...) capable d'hydrolyser en tout ou partie les fonctions amide présentes, et de minimiser ainsi les interactions réticulantes à l'origine de la gélification pour aboutir à des formes solubles.

Sans être lié par la théorie, on peux supposer que les bases ajoutées coupent les chaînes latérales pour laisser de la poly(N-acryloyl glycine) et probablement des copolymères (poly(acide acrylique-co-N-acryloyl glycinamide-co-N-acryloyl glycine) ou encore poly(acide acrylique-co-N-acryloyl glycine) solubles, voire du poly(acide acrylique), sous forme de sel.

Ainsi, l'exemple 3 ci-après atteste de ce phénomène qui peut être mis à profit pour ajuster le temps de présence souhaité sur site dans l'organisme et/ou sur la plaie.

La formation de macromolécules solubles sur site est particulièrement intéressante si leurs masses molaires moyennes sont suffisamment faibles pour qu'elles puissent être éliminées par filtration glomérulaire au niveau des reins.

Comme mentionnée précédemment, l'association décrite ci-dessus se présente sous la forme d'une formulation aqueuse comprenant le polymère sous forme solution (sol) lorsqu'elle se trouve à une température supérieure à la température de transition gel-sol du mélange considéré.

Une solution aqueuse de PAG chaude à une température de quelques degrés supérieure à la température du site d'administration met très peu de temps pour passer de l'état sol à l'état gel lorsqu'elle est mise en contact avec le corps humain, notamment entre 10 secondes et 5 minutes, plus particulièrement entre 30 secondes et 3 minutes.

La solution peut être préparée de manière ex-temporanée, gélifiée et stérilisée localement par ultrafiltration à chaud après fluidification par réchauffement juste avant application si nécessaire ou préféré.

Alternativement, le mélange peut être préparé à l'avance sous forme déshydratée après lyophilisation d'une solution chaude stérilisée ou non en vue de sa conservation. Après réchauffement, la solution comprenant le polymère et le principe actif peut être stérilisée par ultrafiltration si nécessaire et conditionnée après lyophilisation par exemple en vue de sa conservation. La stabilité chimique du polymère conforme à la présente invention offre une possibilité de stockage long sans précaution particulière, ce qui n'est pas le cas des systèmes matriciels dégradables à base de PLA ou de copolymères PLA-PEG notamment.

Ainsi, la présente invention décrit également un lyophilisat préparé à partir d'une solution aqueuse de poly(N-acryloyl glycinamide), et éventuellement d'au moins un principe actif et/ou au moins un produit visible en imagerie médicale et éventuellement d'un additif.

La présente invention décrit également le procédé de préparation d'un lyophilisat tel que défini ci-dessus caractérisé en ce qu'il comprend au moins les étapes suivantes :

- la préparation d'une solution aqueuse gélifiable de poly(N-acryloyl glycinamide) ;
- la stérilisation de ladite solution ;
- la gélification de ladite solution par refroidissement ;
- la congélation de l'hydrogel ; et
- la sublimation de la glace en vapeur d'eau, sous vide.

La présente invention décrit également un lyophilisat préparé à partir d'une solution de poly(N-acryloyl glycinamide) et d'au moins un principe actif et/ou au moins un produit visible en imagerie médicale.

Ainsi, selon une variante de réalisation, la présente invention concerne un lyophilisat préparé à partir d'une solution aqueuse de poly(N-acryloyl glycinamide), et d'au moins un principe actif et/ou d'au moins un produit visible en imagerie médicale, la dite association de poly(N-acryloyl glycinamide) avec au moins un principe actif et/ou au moins un produit visible en imagerie médicale présentant une température de transition gel-sol comprise entre 30 et 60°C, et étant sous forme liquide au dessus de la température de transition gel-sol et sous forme gel en dessous de la température de transition gel-sol.

La présente invention concerne également le procédé de préparation d'un lyophilisat tel que défini ci-dessus caractérisé en ce qu'il comprend au moins les étapes suivantes :

- la préparation d'une solution aqueuse de poly(N-acryloyl glycinamide) et d'au moins un principe actif et/ou au moins un produit visible en imagerie médicale ;
- la stérilisation de ladite solution ;
- la gélification de ladite solution par refroidissement ;
- la congélation de l'hydrogel ; et

- la sublimation de la glace en vapeur d'eau, sous vide.

## Applications

L'association selon l'invention est particulièrement utile pour la délivrance d'un principe actif, notamment en thérapie humaine et vétérinaire.

A titre d'application visée dans le cadre de la présente invention, on peut notamment citer le traitement local par délivrance contrôlée topique de principe actif.

Plus particulièrement, on peut citer le traitement local complémentaire d'une ablation chirurgicale susceptible de laisser présent des agents pathogènes ou des cellules prolifératives localement ou après migration comme c'est le cas de cellules tumorales résiduelles dans certains cancers.

Ainsi, la délivrance locale de principes actifs au niveau du site tumoral via la cavité intrapéritonéale (ip) expose les cellules cancéreuses résiduelles à des doses plus importantes que celles qui peuvent être obtenues par chimiothérapie systémique tout en minimisant les effets secondaires.

La solution aqueuse conforme à la présente invention peut être aisément injectée, alternativement à l'administration intrapéritonéale, directement dans ou au contact immédiat d'une tumeur pour devenir une phase dépôt, selon la volonté du clinicien utilisateur.

Ainsi, dans le cadre de la présente invention, l'association est plus particulièrement utile pour le traitement local post-opératoire ou per-opératoire, notamment des patients ayant subi une chirurgie de réduction tumorale.

Plus précisément, l'association selon l'invention est utile dans le traitement antitumoral local per-opératoire après chirurgie de réduction tumorale.

Par « traitement local per-opératoire », on entend un traitement topique appliqué autour de la zone du corps animal, et plus particulièrement humain, ayant subi l'ablation d'une partie tumorale.

Par ailleurs, dans le cadre de la présente invention, l'association est utile pour le traitement des plaies, où elle peut être soit directement appliquée sur la plaie soit *via* tout dispositif de protection, notamment un pansement, par exemple par imprégnation dudit dispositif de protection par trempage dans la formulation aqueuse sous forme de solution chaude et refroidissement conduisant à un pansement enrobé de gel.

En cas de nécessité face à un retrait rendu difficile par une adhésion trop forte, un bref réchauffement du pansement réticent pour repasser le gel en sol peut être exploité en facilitant le décollement du pansement.

Selon un autre aspect de l'invention, l'association est également utile pour le suivi post-opératoire en imagerie médicale.

L'association selon l'invention peut être utile pour le traitement des animaux, en particulier des humains.

L'association selon l'invention peut également être utile dans de nombreux autres domaines où des formes à libération prolongée de composés actifs sont exploitées, comme en cosmétique (crèmes dépilatoires, agents acide d'érosion cutanée, ...) ou en agriculture (traitement de plantes, des ruches, ...).

L'administration peut mettre en jeu une formulation galénique sous forme de solution gélifiable *in situ.* La même formulation peut également être administrée sous forme de poudre issu d'une lyophilisation, notamment telle que décrite ci-dessus, cette dernière gonflant par capture de l'eau des fluides biologiques locaux. Il est possible aussi d'administrer l'hydrogel lui-même par injection, pourvu que sa viscosité le permette.

La solution aqueuse conforme à la présente invention peut par exemple être également directement appliquée par badigeonnage.

On peut soit utiliser un instrument (pinceau, spatule, peigne) pour étaler la phase sol sur site, soit injecter la phase sol à l'aide d'une seringue chauffante contrôlée, ou encore disperser une poudre de lyophilisat formulée.

L'objet de la présente invention s'étend à tout support galénique pour application topique comprenant une association conforme à la présente invention.

## Kit et conditionnement

Selon un autre aspect, la présente invention concerne un kit ou ensemble de conditionnement, comprenant un récipient comprenant un gel formulé auquel on ajoutera une quantité de liquide physiologique stérile donnée avant réchauffement pour obtenir une solution chaude.

Par « stérile », on entend qualifier un environnement apte à garantir l'innocuité requise.

Alternativement, le kit peut comprendre au moins deux récipients distincts ou deux compartiments distincts d'un même récipient, l'un comprenant un lyophilisat préparé à base de poly(N-acryloyl glycinamide), et l'autre comprenant une quantité suffisante de liquide physiologique stérile permettant de reconstituer le mélange initial par mélange avec ledit lyophilisat et réchauffement pour obtenir une solution chaude.

L'utilisateur peut ainsi reconstituer une composition selon l'invention, en mélangeant de façon extemporanée, juste avant l'application, les différents constituants du kit.

Par exemple, selon un mode de réalisation particulier, on peut disposer d'une poche qui est polycompartimentée, et plus particulièrement bicompartimentée.

Ainsi, la poche peut présenter au moins deux compartiments isolés l'un de l'autre par une membrane étanche mais suffisamment fragile pour se déchirer sous l'action d'une compression, chaque compartiment étant destiné à stocker au moins un composé utile à la fabrication d'une solution conforme à l'invention.

Selon une première alternative, la poche est bicompartimentée, le premier compartiment comprenant au moins du sérum physiologique stérile, et le deuxième compartiment comprenant au moins un lyophilisat de poly(N-acryloyl glycinamide).

Selon une seconde alternative, la poche est tricompartimentée, chaque compartiment pouvant être dédié respectivement au conditionnement du lyophilisat de poly(N-acryloyl glycinamide), du sérum physiologique stérile et d'au moins un principe actif à délivrer et/ou d'au moins un produit visible en imagerie médicale.

Dans ces modes de réalisation dans lesquels les différents composés peuvent avantageusement être isolés l'un de l'autre au sein de compartiments respectifs, une première étape implique alors de réaliser au préalable une rupture de la membrane séparant les compartiments, par exemple au moyen d'une simple pression.

Selon encore un autre aspect, la présente invention concerne un flacon, comprenant au moins un mélange préparé à partir d'une solution aqueuse de poly(N-acryloyl glycinamide) et éventuellement d'au moins un principe actif et/ou d'au moins un produit visible en imagerie médicale, et présentant un septum permettant de prélever ledit mélange après réchauffement du flacon, par exemple à l'aide d'une seringue elle-même chauffante ou chauffée.

Les modes de réalisation décrits ci-dessus permettent de s'affranchir de toute étape nécessitant un contact de la composition avec l'environnement extérieur, réduisant par conséquent significativement les risques de contamination.

Les exemples ci-après illustrent la présente invention sans en limiter la portée.

## Exemple 1

Synthèse du monomère Acryloyl glycinamide (AG)

Dans un réacteur en verre, plongé dans un bain de glace, muni d'une agitation mécanique (à 500 tours/minute) et d'un thermomètre, 113,62 grammes (1,03 mol) de chlorhydrate de glycinamide sont dispersés dans 700 ml de dichlorométhane.

Lorsque la température atteint environ 5 °C, 100 ml de chlorure d'acryloyle (1,23 mol) sont additionnés rapidement. 565 ml d'une solution de carbonate de potassium 2M sont ensuite additionnés goutte à goutte de telle sorte que la température n'excède pas 20 °C. A la fin de l'addition, le pH de la phase aqueuse est environ 7-8 (mesure au papier pH). L'agitation est maintenue pendant 2 à 3 heures à température ambiante.

La phase aqueuse est séparée, passée sur charbon actif, filtrée et immédiatement mise à lyophiliser. 290 g d'un mélange essentiellement constitué d'AG et de chlorure de potassium KCl sont récupérés et broyés finement.

L'AG est extrait de ce mélange avec 16 fractions de 500 ml d'acétone, chaque fraction est placée 5 à 15 minutes dans une cuve à ultrasons.

Dans une première expérience, 93,7 g d'AG brut sont récupérés (le rendement brut est de 70%) et recristallisés dans 3 litres d'acétone. Après lavage à l'acétone, l'AG est séché sous vide (< 0,01 mbar) à 35°C pendant deux jours.

Le rendement de recristallisation est de 68%.

Dans une seconde expérience, les 93,7 g d'AG brut sont récupérés (le rendement brut est de 70%) et recristallisés dans 800 mL d'un mélange acétone - méthanol (9:1 en volume). Après lavage à l'acétone, l'AG est séché sous vide (< 0,01 mbar) à 35°C pendant deux jours.
Le rendement de recristallisation est de 20%.
RMN $^1H$ ($D_2O$): $\delta$(ppm) 6,06 (2H, $CH_2$=C); 5,60 (1H, CH=C), 3,76 (2H,$CH_2$CO).
RMN $^{13}C$ ($D_2O$): $\delta$(ppm) 175,5 (CO-$NH_2$); 169,0 (CO-NH); 129,9 ($CH_2$=CH); 128,6 (CH=$CH_2$); 42,5 ($CH_2$NH).
Analyse calculée pour $C_5H_8N_2O_2$: C, 46,87; H, 6,29; N, 21,86; O, 24,97.
Analyse mesurée pour $C_5H_8N_2O_2$: C, 46,96; H, 6,29; N, 21,74; O, 25,01.
Point de fusion: 126-127°C.

Polymérisation de l'N-acryloyl glycinamide (AG)

La polymérisation radicalaire de l'AG en solution aqueuse est réalisée à concentration en monomère AG et en amorceur $K_2S_2O_8$ constantes: [AG] = 0,5 mol/L et [$K_2S_2O_8$] = 5 x $10^{-4}$ mol/L. La concentration en 2-propanol (agent de transfert = AT) varie entre 0 et 4 mol/L.

L'AG, le $K_2S_2O_8$ (Amorceur redox), l'AT et l'eau sont introduits dans un tube en verre de 150 mL. Le milieu réactionnel est dégazé en faisant circuler un courant d'Argon pendant environ 1 heure. Le tube sous argon est placé

dans un bain d'eau thermostaté à 60 + 0,2 °C pendant 24 à 26 heures.

Deux modes de récupération du poly(acryloyl glycinamide) (PAG) sont utilisés en fonction de la concentration en AT:

1) Pour une concentration [AT] > 1,5 mol/L, le mélange réactionnel est dialysé contre de l'eau à l'aide d'une membrane Spectrapore (pouvoir de coupure $M_W$ = 3500) et lyophilisé ;
2) Pour une concentration [AT] < 1,5 mol/L, le PAG est précipité dans 6 volumes de méthanol à l'aide d'un mixeur (Waring Blendor), puis lavé au méthanol et séché sous vide à 50°C pendant 2 à 3 jours.

Les rendements sont compris entre 80 et 90%.
RMN $^1H$ ($D_2O$-NaSCN 2M): $\delta$ (ppm) 4,00 (2H, $CH_2$-CO); 2,32 (1H, CH-C); 1,72 (2H, $CH_2$-C).
RMN $^{13}C$ ($D_2O$-NaSCN 2M): $\delta$ (ppm) 177,8 (CO-$NH_2$); 174,5 (CONH); 134,0 (SCN); 42,9 ($CH_2$-NH); 35,9 (2C, $CH_2CH$).

Les polymères obtenus sont caractérisés par $^1H$-RMN et $^{13}C$-RMN, par exemple avec un spectromètre Brucker (100 MHz). Les points de fusion sont mesurés sur un banc Kofler. Les mesures viscosimétriques sont réalisées à 25°C sur un appareil automatique du type Ubbelhode ou un viscosimètre automatique.

Le tableau 1 ci-dessous présente des données relatives à la polymérisation radicalaire de l'AG ([AG] = 0,5 mol/L) en solution aqueuse à 60°C amorcée par le persulfate de potassium ([$K_2S_2O_8$] = $5x10^{-4}$ mol/L) pour différentes concentrations en agent de transfert (2-propanol).

Tableau 1

| PAG N° | [2-propanol] en mol/L | [$\eta$] (dl/g) $H_2O$-NaSCN 2M à 25°C | Mn x $10^{-3}$ |
|---|---|---|---|
| 1 | 0 | gel chimique | ---- |
| 2 | 0,16 | 1,12 | 549 |
| 3 | 0,30 | 0,91 | 369 |
| 4 | 0,43 | 0,69 | 216 |
| 5 | 0,69 | 0,62 | 176 |
| 6 | 0,98 | 0,42 | 83 |
| 7 | 1,20 | 0,40 | 79 |
| 8 | 1,66 | 0,28 | 38 |
| 9 | 2,00 | 0,26 | 33 |
| 10 | 3,33 | 0,20 | 20 |
| 11 | 3,99 | 0,18 | 16 |

La synthèse des PAG N°5, 6 et 9 ont été reproduites avec les mêmes conditions opératoires. Les PAG obtenus possèdent les viscosités respectives de 0,55, 0,39 et 0,29 dl/g correspondant respectivement des Mn viscosimétriques de 140, 72 et 41 x $10^{-3}$.

Ces duplications montrent que les écarts sont de l'ordre de 10 à 15%. Ces valeurs sont tolérables compte-tenu de la précision des déterminations de masses molaires moyennes par viscosimétrie.

L'équation suivante *(selon* C.A. Barson in Comprehensive Polymer science, Vol. 3, Chain Polymerization I ; Pergamon Press, New-York, 1989, p. 173*)* peut être utilisée pour déterminer la constante de transfert au 2-propanol :

$$1/DP_n = 1/DP_0 + C_M + C_S[S]/[M] + C_A[A]/[M] + C_{AT}[AT]/[M]$$

Où:

- $DP_0$ représente le degré de polymérisation moyen en nombre $DP_n$ en absence de réactions de transfert;
- $C_M$, $C_A$, $C_S$ et $C_{AT}$ sont les constantes de transfert au monomère M, à l'amorceur A, au solvant S et à l'agent de transfert AT ;
- [S] = la concentration en solvant;
- [A] = la concentration en amorceur ;
- [M] = la concentration en monomère ; et

- [AT] = la concentration en agent de transfert.

La figure 1 représente la variation du degré de polymérisation moyen en nombre $DP_n$ des PAG obtenus à 60°C (□) et à 75°C (■) en fonction de la concentration en 2-propanol (agent de transfert : AT). La concentration en monomère M est constante : [M] = 0,5 mol/L (□) et 0,75 mol/L (■).

Lorsque les concentrations en monomère M et en amorceur A sont constantes et que l'on néglige la faible variation de concentration en solvant par rapport à la concentration en AT (34,5 < $[H_20]$ (mol/L) < 54,5), la constante de transfert au 2-propanol peut être estimée en suivant la variation linéaire de $1/DP_n$ en fonction du rapport [AT]/[M].

Cette variation est représentée sur la figure 1 dans le cas des PAG obtenus, décrits dans le tableau ci-dessus, à 60°C et des PAG obtenus à 75°C.

Les $DP_n$ sont calculés à partir des mesures viscosimétriques dans $H_2O$-NaSCN 2M à 25°C.

Le NaSCN est destructeur de liaisons hydrogène, il permet d'éviter la gélification à température ambiante, afin d'effectuer la caractérisation.

Les points expérimentaux sont dispersés autour d'une droite. La moyenne d'équation respective est :

$1/DP_n$ = 1,02 [AT]/[M] - 3,94 x $10^{-1}$ (Le coefficient de corrélation est $R^2$ = 0,988) à 60°C ; et
$1/DP_n$ = 1,04 [AT]/[M] - 4,94 x $10^2$ (Le coefficient de corrélation est $R^2$ = 0,999) à 75°C.

L'ordonnée à l'origine tend vers zéro et suggère un $DP_0$ très élevé, supérieure à $10^5$.

Les pentes de ces droites permettent d'estimer la constante de transfert du 2-propanol : $C_{AT}$ = 1,02 x $10^{-3}$ à 60°C et $C_{AT}$ = 1,04 x $10^{-3}$ à 75°C.

Ces valeurs sont du même ordre de grandeur que la constante de transfert du 2-propanol dans le cas de la polymérisation radicalaire en solution aqueuse à 50°C de l'acrylamide : $C_{AT}$ = 1,9 x $10^{-3}$.

La température de transition gel-sol des gels de PAG dans l'eau a été déterminée de la façon suivante:

En partant de poudre de PAG sec, 10 g de suspensions à des concentrations C de 2, 4, 6, 8 et 10% en poids dans l'eau sont préparés dans des tubes à essai de 20 ml.

Le phénomène de gélification étant sujet à hystérésis, afin d'obtenir un gel reproductible, on procède à plusieurs cycles gel-sol selon la procédure de recyclage suivante :

Les suspensions sont chauffées à 80°C pendant 2 heures puis refroidies à température ambiante.

La phase sol est reformée par chauffage à 80°C pendant 5 minutes.

Une première température de transition gel-sol est déterminée.

Le sol est alors refroidi à 0°C dans de l'eau et de la glace.

Après une durée de 30 minutes, le gel est ramené lentement, à une vitesse de 4°C/min, à 80°C.

Une nouvelle température de transition gel-sol est enregistrée.

Le gel est de nouveau refroidi à 0°C pendant 30 min.

Deux nouveaux cycles similaires sont engagés.

Les quatre températures observées sont indiquées pour chaque polymère et concentration dans le tableau 2. La reproductibilité apparaît bonne à partir du troisième cycle, parfois dès le deuxième.

Le tableau 2 ci-dessous présente l'influence de cycles chaud-froid successifs sur la température de transition gel-sol $T_{gel/sol}$, sur des formulations de concentration C en PAG de masse molaire Mn.

La masse molaire Mn est déterminée par viscosimétrie à 25°C dans l'eau en présence de NaSCN 2M, en appliquant la loi de viscosité [η] = 1,16 x $10^3$ $Mn^{0,52}$ selon H.C. Hass, R.I. Mac Donald et A.N. Schueler, J. Polym. Sci., Part A1, 1970, 8, 1213.

Tableau 2 (Sur 4 cycles)

| Concentration C (% massique) | Mn = 549 x $10^3$ (100 $cm^3$/g) | Mn = 176 x $10^3$ (100 $cm^3$/g) | Mn = 79 x $10^3$ (100 $cm^3$/g) | Mn = 41 x $10^3$ (100 $cm^3$/g) |
|---|---|---|---|---|
| 2 | **59/61/57/57** | a) | a) | a) |
| 4 | 64/75/70/70 | **52/57/50/50** | a) | a) |
| 6 | 80/80/80/80 | **59/64/62/62** | **35/50/48/49** | **32/36/35/35** |
| 8 | b) | 67/72/70/68 | **55/60/58/58** | **46/52/48/48** |
| 10 | b) | 70/75/75/73 | 60/63/64/63 | **52/56/55/55** |

a) pas de formation de gel à 25°C et à 0°C

b) pas déterminé

On peut déduire du tableau 2 que les PAG de Mn = 549 x $10^3$ à C = 2%, de Mn= 176 x $10^3$ à C = 4% ou 6%, de Mn = 79 x $10^3$ à C = 6% ou 8%, et de Mn = 41 x $10^3$ à C = 6 ou 8% ou 10%, seront particulièrement considérées dans le cadre de la présente invention (parties grisées).

La figure 2 représente la variation de la température de transition gel-sol $T_{gel\text{-}sol}$ en fonction de la concentration pour les PAG de masses moléculaires Mn x $10^{-3}$ = 41 (■), 76 (♦), et 549 (●).

La figure 3 représente une variation des températures de séparation de phase (transition gel-sol) en fonction de la masse moléculaire Mn des PAG pour des concentrations C de 6% (■), 8% (▲) et 10% (●).

**Exemple 2**

Quatre souris normales 1 à 4, traitées selon les règles éthiques en vigueur, ont été opérées par ouverture de la cavité péritonéale à titre préliminaire et ont reçu 1 ml de solution (1, 2, 3 ou 4) vierge associant un PAG (369 000 g/mol) et de la doxorubicine, administrée à l'aide de seringues chauffées à l'air chaud.

Préparation de la solution 1 pour la souris 1 : 100 mg de PAG N°3 de l'exemple 1 en poudre sont placés dans une fiole d'Erlenmeyer. 4 ml de sérum physiologique y sont ajoutés. Le mélange est chauffé progressivement pour atteindre une température de dissolution avec la formation d'un milieu homogène à 60-65°C. Le mélange refroidi mais demeurant en solution, est administré dans la cavité intrapéritonéale de la souris 1 et laissé gélifié sur site pendant 2 minutes jusqu'à gélification à 37°C. Le ventre de la souris est refermé par quelques points.

Préparation de la solution 2 pour la souris 2 : La solution 2 est préparée selon le même mode opératoire que la solution 1 avec les quantités suivantes : 25 mg de PAG N°3 et 4 mg de Doxorubicine chlorhydrate (Dox) dans 4ml de sérum physiologique sont introduits après la formation du milieu homogène.

Préparation de la solution 3 pour la souris 3 : La solution 3 est préparée selon le même mode opératoire que la solution 1 avec les quantités suivantes : 100 mg de PAG N° 3 et 4 mg de Doxorubicine chlorhydrate (Dox) dans 4 ml de sérum physiologique sont introduits après la formation du milieu homogène.

Préparation de la solution 4 pour la souris 4 : La solution 4 est préparée selon le même mode opératoire que la solution 1 avec les quantités suivantes : 100 mg de PAG N° 3 et 0,8 mg de Doxorubicine chlorhydrate (Dox) dans 4 ml de sérum physiologique sont introduits après la formation du milieu homogène (concentration thérapeutique).

Lors de l'administration du mélange non gélifiant dans la souris 2, l'opérateur a constaté une fuite de la solution lors de la fermeture de la cavité, inconvénient bien connu des cliniciens lorsqu'ils utilisent des solutions d'agent antitumoral.

Après deux jours, les souris 2 et 3, vivantes mais très affectées par la forte dose en Dox, ont été sacrifiées. La cavité intrapéritonéale de la souris 2 est apparue vide. La souris 3 est apparue quasiment normale avec seulement des traces de gel sous forme de petits morceaux calés dans les infractuosités.

Il semble qu'une large partie du gel ait disparu au moins visuellement.

Les deux autres souris 1 et 4 étaient toujours en vie au bout de 4 jours et aucune ne semblait être affectée par la présence du gel ou du gel-Dox.

**Exemple 3 : Mélanges pour test de dégradabilité partielle de PAG à PAA (poly(acide acrylique)) salifié**

40 mg de PAG N°3 sont mis en présence de 1 ml d'eau dans un flacon chauffé à 80°C pour avoir une solution limpide, la solution est gélifiée par refroidissement et subit deux nouveaux cyclages chaud-froid (comme décrit dans l'exemple 1) pour obtenir un gel homogène.
4,5 mg d'un minéral finement pulvérisé ($BaSO_4$, $Ca(OH)_2$, $Mg(OH)_2$ ou $Na_2CO_3$) sont ajoutés et le mélange gel-minéral est remis en solution à chaud et la dispersion est homogénéisée à l'aide d'un homogénéïseur et refroidie sous agitation pour fournir une dispersion homogène de sel dans le gel après refroidissement.
Les différents flacons sont ensuite placés dans un incubateur porté à 37°C.
Selon le minéral, on observe :

- Pour le $BaSO_4$ (Sulfate de baryum) une opacité du gel et une stabilité du gel sur 5 semaines ;
- Pour le $Ca(OH)_2$ (Hydroxyde de calcium) qui est une base faible très peu soluble, le milieu est toujours gélifié après 5 semaines ;
- Pour $Mg(OH)_2$ (Hydroxyde de magnésium) qui est une base faible peu soluble, le gel perd de sa viscosité après 4 jours. Après 2 semaines, le gel a fait place à une solution très visqueuse ; et
- Pour $Na_2CO_3$ qui est une base plus forte, le gel se liquéfie dès le deuxième jour et laisse progressivement place à une solution visqueuse et limpide.

Cet exemple vise à illustrer la modulation pouvant être mise en oeuvre en vue d'ajuster la « dégradabilité solubilisante » des PAG considérés.

**Exemple 4 : Association comprenant un agent opacifiant**

Le même protocole que pour les produits basiques de l'exemple 3 est utilisé pour obtenir un gel chargé à 10% en poids du sulfate de baryum ou d'oxyde de zirconium.
Une telle concentration de ces agents est utilisée afin de rendre les ciments orthopédiques opaques aux rayons X.
Les hydrogels correspondants sont alors détectables par rayons X ce qui permet de suivre leur devenir *in situ.*

**Exemple 5 : Association comprenant des nanoparticules paramagnétiques**

Le même protocole que pour les exemples 3 et 4 est appliqué à 100 $\mu$l de sol et 2 mg de nanoparticules paramagnétiques (NPM) de 5 à 200 nm de type oxyde de fer $Fe^{2+}/Fe^{3+}$ sont introduites dans le mélange, afin de rendre le sol et le gel sensibles aux sollicitations magnétiques utilisables pour donner une forme lors de la gélification ou encore de modifier la libération d'un composé piégé temporairement au sein d'un tel gel.
Après homogénéisation lors de la gélification, le gel coloré brun orangé est parfaitement stable.
Après 10 semaines, l'ajout de 2 ml d'eau n'affecte pas son comportement paramagnétique et la solution surnageante reste incolore pendant au minimum 4 mois, ce qui confirme le piégeage des NPM.
On constate que l'association conforme à la présente invention présente un avantage en terme de stabilité.

**Exemple 6 : Association de PAG avec des principes actifs**

La libération de trois composés (« modèles de drogue ») a été testée indépendamment :

1- L'acétate de cobalt ;
2- Le poly(acrylate de sodium) fluorescent de masse moléculaire égale à 2000-2500 g/mol ; et
3- L'oligomère de poly(tyrosine-formaldéhyde) de masse moléculaire égale à 2600 g/mole.

Mode opératoire de préparation:

60 mg de polymère PAG de masse moléculaire égale à 170000 g/mol sont mis en présence de1 ml de sérum physiologique (6% en masse) dans un petit flacon cylindrique fermé hermétiquement et chauffé à 60°C pendant quelques minutes pour obtenir une solution homogène et limpide qui est gélifiée par refroidissement.
Le cycle est répété deux fois pour parfaire l'homogénéité du gel. 10 mg du composé choisi comme modèle de drogue sont ajoutés. Le mélange est réchauffé pour avoir une solution homogène qui est laissée à refroidir pour obtenir le système composé-gel.
Le flacon est placé dans un incubateur à 37°C mobile après avoir ajouté au dessus du gel formé 1 ml de sérum

physiologique où le composé piégé au sein de l'hydrogel va diffuser progressivement. Le milieu de réception est alors renouvelé à des temps réguliers et le composé ayant diffusé est évalué quantitativement par spectrométrie UV. La courbe de libération progressive est tracée en pourcentage de la quantité initialement placée dans le gel.

1) Exemple de l'acétate de cobalt, sel minéral :

La figure 4 représente la courbe de libération d'acétate de cobalt dans le poly(N-acryloyl glycinamide) de masse molaire moyenne égale à 170000 g/mol, en fonction du temps.

Cette courbe est remarquable par un faible « *burst* » (inférieur à 15% à 1 heure) et par une libération progressive allant jusqu'à épuisement complet. Le « *burst* » est une libération à très court terme due au passage en solution du principe actif disponible en surface, c'est-à-dire pas retenu par la matrice.

Dans le même milieu sans gel, le sel de cobalt sans gel se dissout instantanément dans sa totalité.

2) Exemple d'un poly(acrylate de sodium) marqué au niveau de 2% des unités de répétition par de la fluorescéinamine couplée de manière covalente (PAAFLU) :

La figure 5 représente la courbe de libération du poly(acrylate de sodium) dans le poly(N-acryloyl glycinamide) de masse molaire moyenne égale à 170000 g/mol, en fonction du temps.

Ici encore, le « *burst* » est très faible (inférieur à 10%) et la libération progressive va jusqu'à épuisement.

3) Exemple d'un oligomère de poly(tyrosine-formaldéhyde) dit MV de masse moléculaire égale à 2600 /g mol :

La figure 6 représente la courbe de libération de l'oligomère de poly(tyrosine-formaldéhyde) dans le poly(N-acryloyl glycinamide) de masse molaire moyenne égale à 170000 g/mol, en fonction du temps.

Ici encore, le « *burst* » est faible et la libération est totale.

**Exemple 7 : Injection sur souris avec le bleu de méthylène comme modèle de drogue anti-tumorale pour administration intrapéritonéale**

On observe la délivrance du colorant de l'hydrogel chargé vers les tissus et organes de la cavité intrapéritonéale chez la souris.

Pour cela, une solution isotonique de PAG (de masse molaire égale à environ 176000 g/mol, 6% en poids de PAG) contenant du bleu de méthylène (4 ml de solution (20 mg de colorant) pour 16 ml de gel) formée à 60°C conditionnée est introduite dans des seringues chauffées pour être injectée rapidement à 45°C à travers une aiguille de 23 gauges afin de remplir la cavité intrapéritonéale des souris.

Les souris traitées selon les procédures réglementaires ont été sacrifiées après 2 h, 8 h, 24 h, 30 et 52 h (deux animaux par point).

Après ouverture de l'abdomen, les distributions de la couleur bleu au niveau du gel, des tissues et des organes ont été appréciées visuellement et comparées aux mêmes temps avec celles observées pour les souris témoins ayant reçu par injection similaire une solution isotonique de même concentration en bleu de méthylène.

Sur les souris traitées, la présence retardée du colorant a été observée au niveau du gras des tissus intestinaux et abdominaux avec une coloration persistante au niveau du peritoneum et des organes abdominaux encore détectable après 52 heures, le gel se décolorant progressivement. En effet, le gel, les tissues et les organes, notamment le foie, sont demeurés colorés pendant au moins 52 heures.

Sur les souris de contrôle, la couleur bleue a totalement disparue après 24h.

Ces résultats montrent qu'un gel PAG injecté donne bien lieu à un effet retard *in vivo* et qu'il peut être exploité pour la libération progressive d'une molécule modèle piégée temporairement dans la cavité intrapéritonéale.

## Revendications

1. Association de poly(N-acryloyl glycinamide) avec au moins un principe actif et/ou au moins un produit visible en imagerie médicale, dans un milieu aqueux physiologiquement acceptable, ladite association présentant une température de transition gel-sol comprise entre 30 et 60°C, et étant sous forme liquide au dessus de la température de transition gel-sol et sous forme gel en dessous de la température de transition gel-sol, pour son utilisation dans le traitement local post-opératoire ou per-opératoire,
   pour son utilisation dans le traitement des plaies, **caractérisée en ce qu'**elle est directement appliquée sur la plaie ou *via* tout dispositif de protection, ou

pour son utilisation dans le suivi post-opératoire en imagerie médicale.

2. Association pour son utilisation selon la revendication 1, **caractérisée en ce que** ledit poly(N-acryloyl glycinamide) présente une masse molaire moyenne comprise entre 10 000 et 1 000 000 g/mol, en particulier entre 30 000 et 600 000 g/mol, voire entre 40 000 et 200 000 g/mol.

3. Association pour son utilisation selon la revendication 1 ou 2, **caractérisée en ce qu'**elle présente une température de transition gel-sol comprise entre 38 et 50°C, par exemple mesurée par la méthode du tube renversé.

4. Association pour son utilisation selon l'une quelconque des revendications précédentes, **caractérisée en ce que** ledit poly(N-acryloyl glycinamide) est présent dans le milieu aqueux dans une teneur en matière sèche comprise entre 0,5 et 20 % en poids, par rapport au poids total dudit milieu, en particulier dans une teneur comprise entre 1 et 10 % en poids, voire entre 2 et 8 % en poids.

5. Association pour son utilisation selon l'une quelconque des revendications précédentes, **caractérisée en ce que** ledit milieu aqueux est préparé à base d'un liquide physiologique.

6. Association pour son utilisation selon l'une quelconque des revendications précédentes, **caractérisée en ce que** ledit principe actif est choisi parmi les agents antitumoraux, les antibiotiques, les analgésiques, les anti-inflammatoires, les facteurs de croissance et les antiseptiques.

7. Association pour son utilisation selon la revendication précédente, **caractérisée** en que ledit principe actif est un agent antitumoral, notamment choisi parmi la doxorubicine, le paclitaxel, le topotécan, et l'irinotécan.

8. Association pour son utilisation selon l'une quelconque des revendications précédentes, **caractérisée en ce que** ledit produit visible en imagerie médicale est choisi parmi un opacifiant, un agent fluorescent ou un émetteur de radiations ionisantes.

9. Association selon l'une quelconque des revendications précédentes, pour son utilisation dans la délivrance d'un principe actif.

10. Association selon la revendication précédente, pour son utilisation dans le traitement local post-opératoire ou per-opératoire des patients ayant subi une chirurgie de réduction tumorale.

11. Association pour son utilisation selon l'une quelconque des revendications précédentes, ledit dispositif de protection étant un pansement.

12. Association pour son utilisation selon l'une quelconque des revendications précédentes, **caractérisée en ce qu'**elle est appliquée par imprégnation dudit dispositif de protection.

13. Association selon l'une quelconque des revendications précédentes, pour son utilisation dans le traitement des animaux, en particulier des humains.

14. Lyophilisat, **caractérisé en ce qu'**il est préparé à partir d'une solution aqueuse de poly(N-acryloyl glycinamide), et d'au moins un principe actif et/ou d'au moins un produit visible en imagerie médicale, la dite association de poly(N-acryloyl glycinamide) avec au moins un principe actif et/ou au moins un produit visible en imagerie médicale présentant une température de transition gel-sol comprise entre 30 et 60°C, et étant sous forme liquide au dessus de la température de transition gel-sol et sous forme gel en dessous de la température de transition gel-sol.

15. Procédé de préparation d'un lyophilisat tel que défini selon la revendication précédente, **caractérisé en ce qu'**il comprend au moins les étapes suivantes :

- la préparation d'une solution aqueuse gélifiable de poly(N-acryloyl glycinamide) et d'au moins un principe actif et/ou d'au moins un produit visible en imagerie médicale ;
- la stérilisation de ladite solution ;
- la gélification de ladite solution par refroidissement ;
- la congélation de l'hydrogel ;
- la sublimation de la glace en vapeur d'eau, sous vide.

**16.** Kit ou ensemble de conditionnement, comprenant, dans au moins deux récipients distincts ou deux compartiments distincts d'un même récipient, d'une part au moins un lyophilisat selon la revendication 14, et d'autre part au moins une quantité suffisante de liquide physiologique.

## Patentansprüche

**1.** Zusammensetzung aus Poly (N-acryloyl Glycinamid) mit wenigstens einem Wirkstoff und / oder wenigstens einem in der medizinischen Bildgebung sichtbaren Produkt in einem physiologisch verträglichen wässrigen Medium, wobei die Zusammensetzung eine Sol-Gel-Übergangstemperatur zwischen 30 und 60 ° C zeigt und oberhalb der Sol-Gel-Übergangstemperatur flüssig und unterhalb der Sol-Gel-Übergangstemperatur gelförmig ist, zur lokalen, post-operativen oder intraoperativen Verwendung, zur Verwendung bei der Wundbehandlung,
**dadurch gekennzeichnet, dass** sie direkt oder über jegliche Schutzvorrichtung auf die Wunde aufgebracht wird oder zur Verwendung bei postoperativen Versorgung in der medizinischen Bildgebung.

**2.** Zusammensetzung zur Verwendung nach Anspruch 1,
**dadurch gekennzeichnet, dass** das Poly (N-acryloyl Glycinamid) ein mittleres Molekulargewicht zwischen 10 000 und 1 000 000 g / mol, insbesondere zwischen 30 000 und 600 000 g / mol, oder sogar zwischen 40 000 und 200 000 g / mol hat.

**3.** Zusammensetzung zur Verwendung nach Anspruch 1 oder 2,
**dadurch gekennzeichnet, dass** sie eine Sol-Gel-Übergangstemperatur zwischen 38 und 50° C hat, beispielsweise gemessen mit der Methode der umgekehrten Röhre.

**4.** Zusammensetzung zur Verwendung nach einem der vorhergehenden Ansprüche,
**dadurch gekennzeichnet, dass** das Poly (N-acryloyl Glycinamid) in dem wässrigen Medium in einem Trockenmassegehalt von zwischen 0,5 und 20 Gewichts-% bezogen auf das Gesamtgewicht des Mediums vorliegt, insbesondere in einem Gehalt von zwischen 1 und 10 Gewichts-% oder sogar zwischen 2 und 8% Gewichts-%.

**5.** Zusammensetzung zur Verwendung nach einem der vorhergehenden Ansprüche,
**dadurch gekennzeichnet dass** wässrige Medium auf der Grundlage einer physiologischen Flüssigkeit hergestellt ist.

**6.** Zusammensetzung zur Verwendung nach einem der vorhergehenden Ansprüche,
**dadurch gekennzeichnet, dass** der Wirkstoff ausgewählt ist aus den Antitumormitteln, Antibiotika, Analgetika, entzündungshemmenden Mitteln, Wachstumsfaktoren und Antiseptika.

**7.** Zusammensetzung zur Verwendung nach dem vorhergehenden Anspruch,
**dadurch gekennzeichnet, dass** der Wirkstoff ein Antitumormittel ist, insbesondere ausgewählt aus Doxorubicin, Paclitaxel, Topotecan und Irinotecan.

**8.** Zusammensetzung zur Verwendung nach einem der vorhergehenden Ansprüche,
**dadurch gekennzeichnet, dass** das in der medizinischen Bildgebung sichtbare Produkt ausgewählt ist aus einem Trübungsmittel, einem Fluoreszenzmittel oder einem Emitter ionisierender Strahlung.

**9.** Zusammensetzung einem der vorhergehenden Ansprüche zur Verwendung für die Abgabe eines Wirkstoffs.

**10.** Zusammensetzung nach dem vorhergehenden Anspruch zur Verwendung für die postoperative oder intraoperative lokale Behandlung von Patienten, die tumorreduzierend operiert wurden.

**11.** Zusammensetzung zur Verwendung nach einem der vorhergehenden Ansprüche,
**dadurch gekennzeichnet, dass** die Schutzvorrichtung ein Verbandmaterial ist.

**12.** Zusammensetzung zur Verwendung nach einem der vorhergehenden Ansprüche,
**dadurch gekennzeichnet, dass** sie durch Tränken der Schutzvorrichtung aufgebracht wird.

**13.** Zusammensetzung zur Verwendung nach einem der vorhergehenden Ansprüche für die Behandlung von Tieren, insbesondere Menschen.

14. Lyophilisat, **dadurch gekennzeichnet, dass** es auf der Grundlage einer wässrigen Lösung von Poly (N-acryloyl Glycinamid) und wenigstens einem Wirkstoff und / oder wenigstens einem in der medizinischen Bildgebung sichtbaren Produkt hergestellt ist, wobei diese Zusammensetzung aus einer wässrigen Lösung von Poly (N-acryloyl Glycinamid) und wenigstens einem Wirkstoff und / oder wenigstens einem in der medizinischen Bildgebung sichtbaren Produkt eine Sol-Gel-Übergangstemperatur zwischen 30 und 60 ° C zeigt und oberhalb der Sol-Gel-Übergangstemperatur flüssig und unterhalb der Sol-Gel-Übergangstemperatur gelförmig ist.

15. Verfahren zur Herstellung eines Lyophilisats nach dem vorhergehenden Anspruch, **dadurch gekennzeichnet, dass** es wenigstens die folgenden Schritte umfasst:

    - Herstellung einer gelbildenden wässrigen Lösung von Poly (N-acryloyl Glycinamid) und wenigstens einem Wirkstoff und / oder wenigstens einem in der medizinischen Bildgebung sichtbaren Produkt;
    - Sterilisation dieser Lösung;
    - Gelbildung der Lösung durch Abkühlen;
    - Einfrieren des Hydrogels;
    - Sublimation des Eises in Wasserdampf unter Vakuum.

16. Kit oder Verpackungsanordnung, umfassend, in mindestens zwei getrennten Behältern oder zwei getrennten Kammern des gleichen Behälters, einerseits wenigstens ein Lyophilisat nach Anspruch 14 und andererseits wenigstens eine ausreichende Menge an physiologischer Flüssigkeit.

**Claims**

1. A combination of poly(N-acryloylglycinamide) with at least one active principle and/or at least one product that is visible in medical imaging, in a physiologically acceptable aqueous medium, said combination having a gel-sol transition temperature of between 30 and 60°C, and being in liquid form above the gel-sol transition temperature and in gel form below the gel-sol transition temperature,
   for its use in the local post-operative or peroperative treatment,
   for its use in the treatment of wounds, **characterized in that** it is applied directly to the wound or *via* any protective device, or
   for its use in the post-operative monitoring by medical imaging.

2. The combination for its use according to claim 1, **characterized in that** said poly(N-acryloylglycinamide) has an average molar mass of between 10 000 and 1 000 000 g/mol, in particular between 30 000 and 600 000 g/mol, or even between 40 000 and 200 000 g/mol.

3. The combination for its use according to claim 1 or 2, **characterized in that** it has a gel-sol transition temperature of between 38 and 50°C, for example measured via the inverted tube method.

4. The combination for its use according to any one of the preceding claims, **characterized in that** said poly(N-acryloylglycinamide) is present in the aqueous medium in a solids content of between 0.5 and 20% by weight, relative to the total weight of said medium, in particular in an amount of between 1 and 10% by weight, or even between 2 and 8% by weight.

5. The combination for its use according to any one of the preceding claims, **characterized in that** said aqueous medium is prepared based on a physiological liquid.

6. The combination for its use according to any one of the preceding claims, **characterized in that** said active principle is chosen from antitumor agents, antibiotics, analgesics, anti-inflammatory agents, growth factors and antiseptics.

7. The combination for its use according to the preceding claim, **characterized in that** said active principle is an antitumor agent, chosen especially from doxorubicin, paclitaxel, topotecan and irinotecan.

8. The combination for its use according to any one of the preceding claims, **characterized in that** said product that is visible in medical imaging is chosen from an opacifier, a fluorescer and an ionizing radiation emitter.

9. The combination according to any one of the preceding claims, for its use for delivering an active principle.

**10.** The combination according to the preceding claim, for its use in the local post-operative or peroperative treatment for patients who have undergone a tumor reduction surgery.

**11.** The combination for its use according to any one of the preceding claims, said protective device being a dressing.

**12.** The combination for its use according to any one of the preceding claims, **characterized in that** it is applied by impregnation of said protective device.

**13.** The combination according to any one of the preceding claims, for its use in the treatment of animals, in particular humans.

**14.** A lyophilizate, **characterized in that** it is prepared from an aqueous solution of poly(N-acryloylglycinamide), and of at least one active principle and/or of at least one product that is visible in medical imaging, said combination of poly(N-acryloylglycinamide) with at least one active principle and/or at least one product that is visible in medical imaging having a gel-sol transition temperature of between 30 and 60°C, and being in liquid form above the gel-sol transition temperature and in gel form below the gel-sol transition temperature.

**15.** A process for preparing a lyophilizate as defined according to the preceding claim, **characterized in that** it comprises at least the following steps:

- the preparation of a gelable aqueous solution of poly(N-acryloylglycinamide) and of at least one active principle and/or of at least one product that is visible in medical imaging;
- sterilization of said solution;
- gelation of said solution by cooling;
- freezing of the hydrogel;
- sublimation of the ice as water vapor, under vacuum.

**16.** A kit or packaging assembly, comprising, in at least two separate containers or two separate compartments of the same container, on the one hand at least one lyophilizate according to claim 14, and on the other hand at least a sufficient amount of physiological liquid.

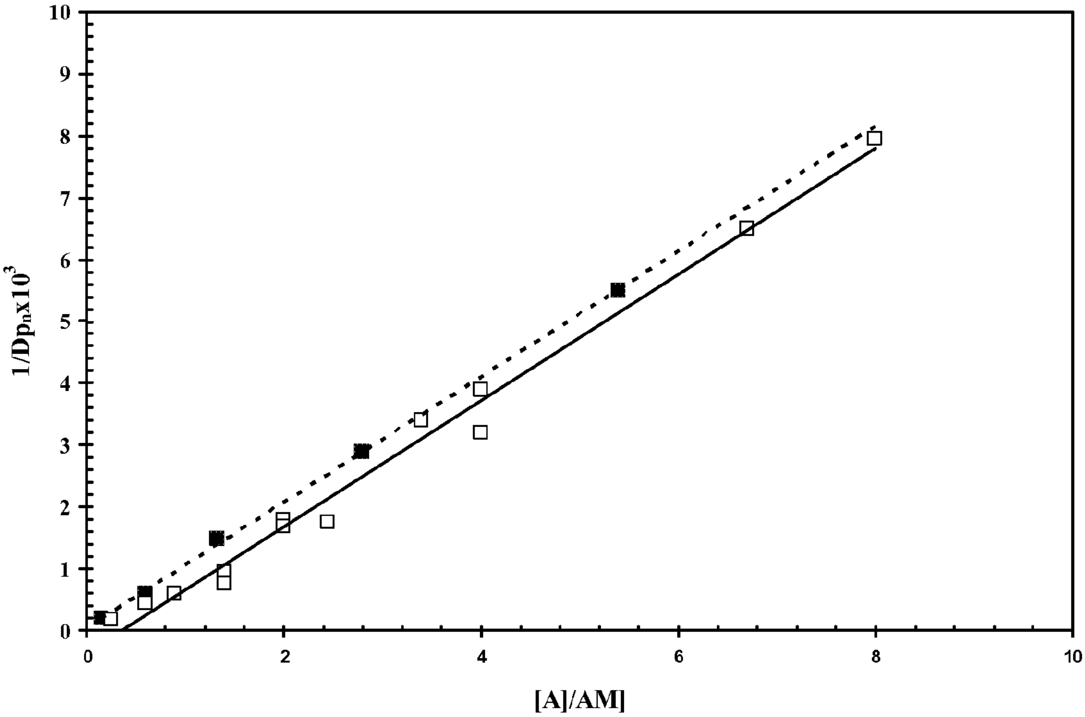


**Figure 1**

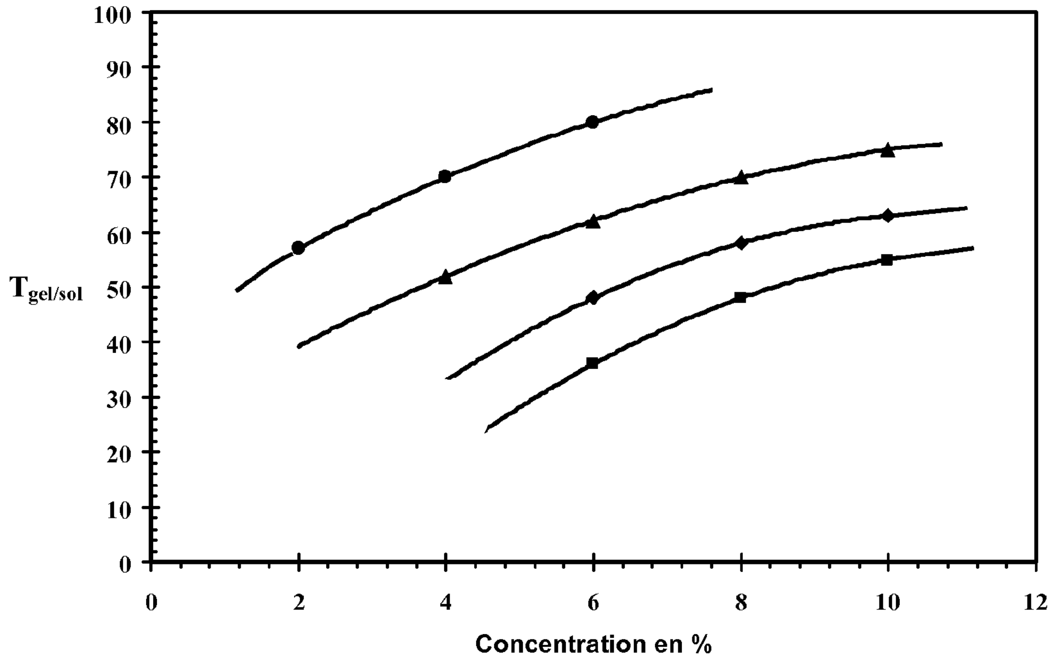


**Figure 2**

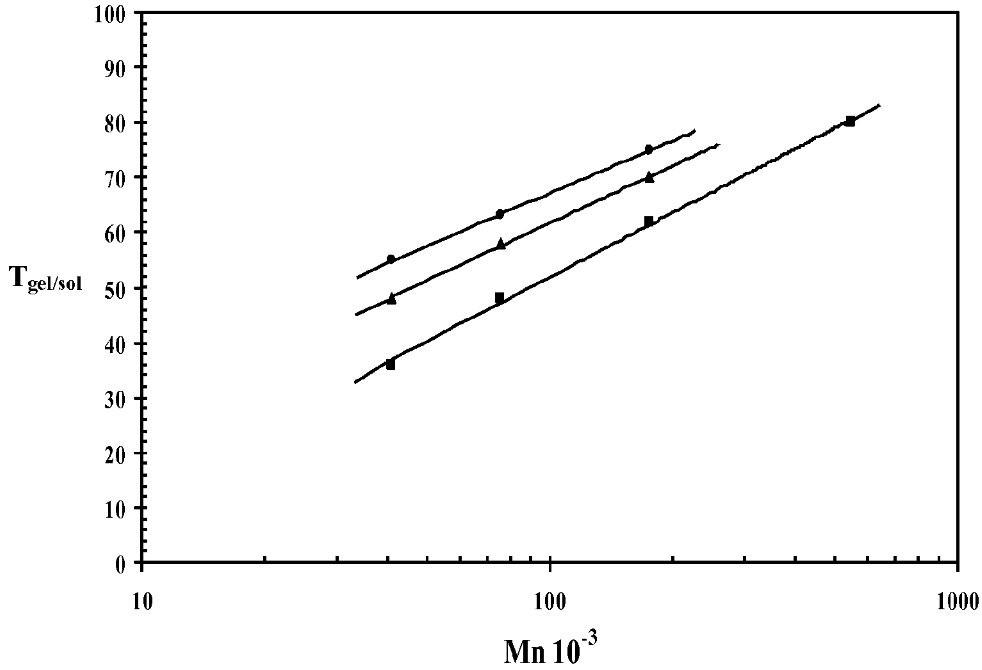


Figure 3

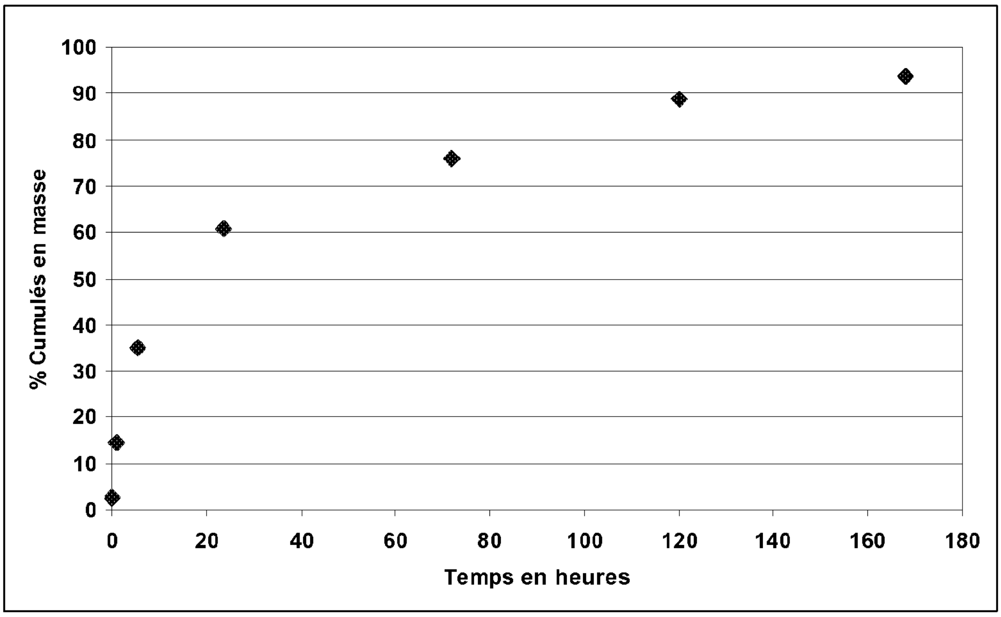


Figure 4

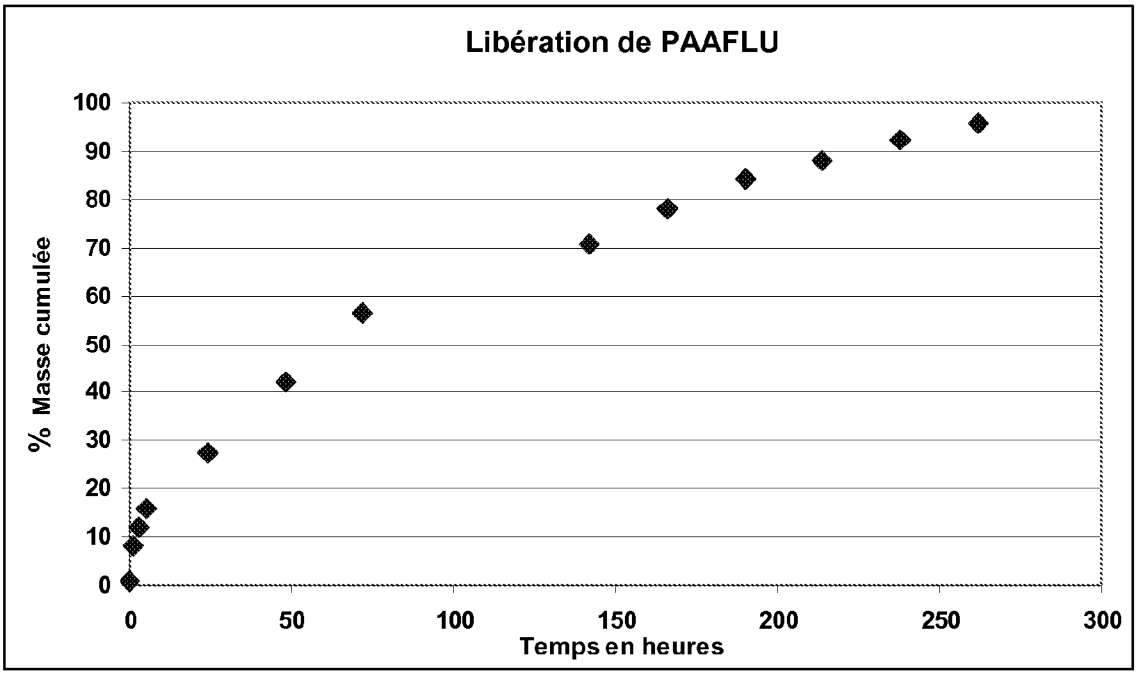


Figure 5

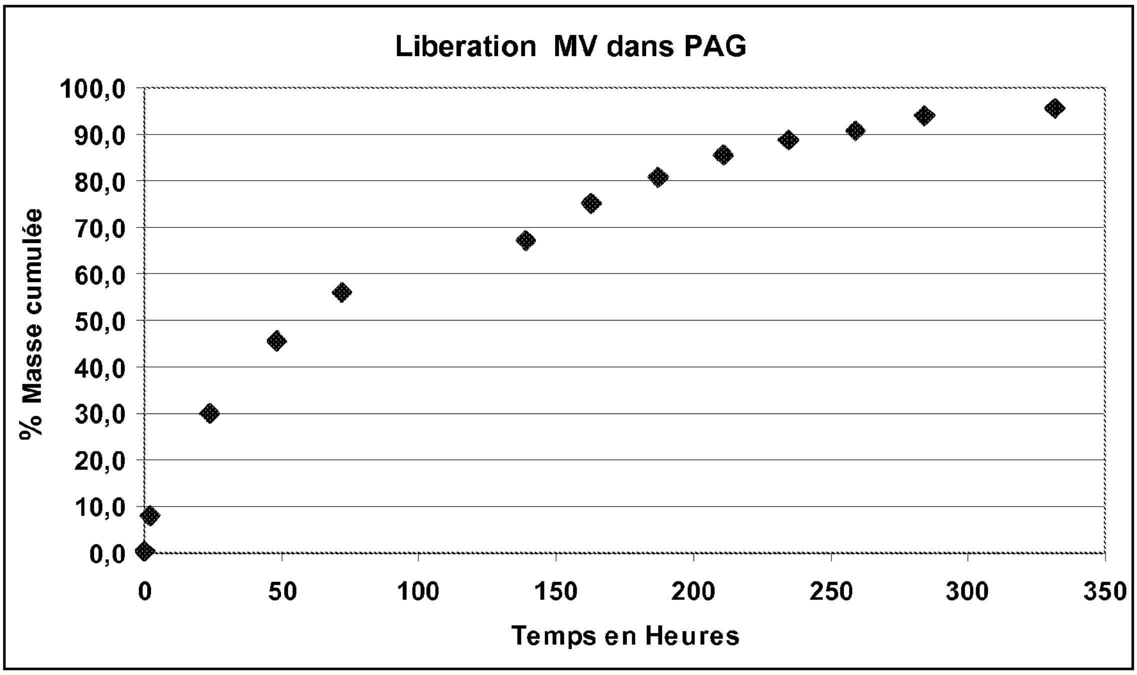


Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20090053276 A
- US 3396030 A


### Littérature non-brevet citée dans la description


- **AGUILAR et al.** Smart Polymers and Their Applications as Biomaterials. *Topics in Tissue Engineering,* 2007, vol. 3
- **HASS et al.** Thermally reversible homopolymer gel systems. *Journal of Polymer Science,* 1964, vol. 2 (12), 1095
- **HAAS et al.** Differential thermal analysis of thermally reversible gels. *Anal. Calorimetry, Proc. Symp.,* 1970, vol. 2, 211
- **KUILIN DENG et al.** *Iranian Polymer Journal,* 2011, vol. 20 (3), 185
- **GLATZEL S. et al.** Well-defined uncharged polymers with a sharp UCST in water and in physiological milieu. *Macromolecules,* 2011, vol. 44, 413
- *Pure and Applied Chemistry,* 2004, vol. 76, 1985
- Definition of terms related to polymer blends, composites, and multiphase polymeric materials. *IUPAC Recommendations,* 2004, 1999
- **HAAS et al.** Synthetic thermally reversible gel systems. *Journal of Polymer Science, Polymer Physics Edition,* 1967, vol. 5 (5), 915
- **HAAS et al.** Synthetic thermally reversible gel systems. *Journal of Polymer Science,* 1970, vol. 8 (5), 1213
- **HAAS et al.** *Journal of Polymer Science,* 1964, vol. 2 (12), 1095
- **GLATZEL et al.** Well-defined synthetic polymer with a protein-like gélation behavior in water. *Chemical Communications,* 2010, vol. 45, 4517
- Comprehensive Polymer science. **C.A. BARSON.** Chain Polymerization I. Pergamon Press, 1989, vol. 3, 173
- **H.C. HASS ; R.I. MAC DONALD ; A.N. SCHUELER.** *J. Polym. Sci.,* 1970, vol. 8, 1213